# EUROPEAN PATENT APPLICATION

(11) **EP 2 647 389 A1**
(43) Date of publication of application: **09.10.2013**
(21) Application number: 12163254.1
(22) Date of filing: 04.04.2012
(51) Int. Cl.: A61K 41/00, A61K 49/18

(54) **Magnetic nanoparticle dispersion, its preparation and diagnostic and therapeutic use**

(71) Applicant: Charité - Universitätsmedizin Berlin, 10117 Berlin (DE); Physikalisch-Technische Bundesanstalt (PTB), 38116 Braunschweig (DE)
(72) Inventor: Kratz, Harald, 10249 Berlin (DE); Wagner, Susanne, 15831 Mahlow (DE); Schnorr, Jörg, 16515 Oranienburg (DE); Taupitz, Matthias, 15831 Mahlow (DE); Ebert, Monika, 15831 Mahlow (DE); Eberbeck, Dietmar, 10435 Berlin (DE)
(74) Representative: Gulde Hengelhaupt Ziebig & Schneider

(57) **Abstract**

The present invention relates to magnetic particle dispersions comprising coated individual monocrystalline and/or polycrystalline nanoparticles of iron oxides and nanoparticulate aggregates (multi-core particles) thereof with improved nonlinear magnetization behavior and improved heating properties in alternating magnetic fields. When measured in a magnetic particle spectrometer (MPS) the particle dispersions show a pronounced overtone structure, especially in the higher harmonics, which surpasses all previously known particle systems many times over. Therefore, the dispersions are especially useful for applications such as MPI (magnetic particle imaging). In addition, the new particle dispersions are suitable for applications in therapeutic hyperthermia, particularly passive partial-body hyperthermia or cell tracking and magnetic resonance imaging (MRI). Hence, the diagnostic and therapeutic use of the dispersions as well as pharmaceutical compositions of diagnostic or therapeutic interest comprising these dispersions are also objects of the present invention.

## Description

The present invention relates to magnetic particle dispersions comprising coated individual monocrystalline and/or polycrystalline nanoparticles of iron oxides and nanoparticulate aggregates (multi-core particles) thereof with improved nonlinear magnetization behavior and improved heating properties in alternating magnetic fields. When measured in a magnetic particle spectrometer (MPS) the particle dispersions show a pronounced overtone structure, especially in the higher harmonics, which surpasses all previously known particle systems many times over. Therefore, the dispersions are especially useful for applications such as MPI (magnetic particle imaging). In addition, the new particle dispersions are suitable for applications in therapeutic hyperthermia, particularly passive partial-body hyperthermia or cell tracking and magnetic resonance imaging (MRI). Hence, the diagnostic and therapeutic use of the dispersions as well as pharmaceutical compositions of diagnostic or therapeutic interest comprising these dispersions are also objects of the present invention.

In the field of technical applications the dispersions can be used for manufacturing electrets, pigments, functional coatings and for instance for final inspection in industrial production of non metal containing parts.

In the prior art a hugh number of different magnetic nanoparticles and aqueous dispersions or suspensions is comprising them is described. The described particles are so-called single-core particles or multi-core particles. For in vivo applications and for stabilization the magnetic particles are coated with a biocompatible shell, preferably with a biocompatible polymer.

The most widely used particles are particles based on magnetic iron oxids. Iron oxides based on magnetite (Fe₃O₄) and/or maghemite (γ-Fe₂O₃) exhibit ferrimagnetic behavior in magnetic fields. If nanoparticles of magnetite (Fe₃O₄) and/or maghemite (γ-Fe₂O₃) fall below a particular size, their behavior is superparamagnetic under certain circumstances, that is, they lack any residual magnetization (remanence) after turning off a previously activated magnetic field. Superparamagnetic iron oxide nanoparticles can be widely used e.g. in magnetic resonance tomography (MRT). The production and use of such particle preparations for use in MRT has been described in US 5,424,419, DE 196 12 001 A1 and DE 4 428 851 A1, for example. But due to the fundamentally different physical phenomena which are used for imaging in the MRT and MPI methods, the suitability of a particle described as a contrast agent for MRT does not determine whether or not the particle is suitable for MPI.

Magnetic particle imaging (MPI) is a new imaging modality allowing direct representation and quantification of superparamagnetic iron oxide nanoparticles (SP10s). The magnetization curve of SPIOs in magnetic fields is nonlinear, making it possible to measure overtones in addition to the incident fundamental frequency in alternating magnetic fields. These signals are specific to SPIOs and thus enable measurement with high sensitivity. Compared to MRT, the method provides potentially higher temporal and spatial resolution and therefore can be used not only in technical applications in the field of plastics, but also in non-invasive medical diagnostics, e.g. in diagnostic of cardiovascular diseases and particularly in the field of coronary heart diseases. Utilizing the potential of MPI requires special tracers exhibiting a particularly pronounced overtone structure in alternating magnetic fields, potentially resulting in high sensitivity of MPI measurements. A magnetic particle spectrometer (MPS) allows measurement of overtones generated by a sample in an alternating magnetic field.

Iron oxide nanoparticle preparations which are suitable for MPI are for instance described in EP 1 738 774 A1. These particles have a diameter of 20 nm to 1µm with an overall particle diameter/core diameter ratio of less than 6. They are coated with a pharmaceutically acceptable polymer which is for instance carboxydextran or PEG. Carboxydextran stabilized iron oxid particles are the particles which are contained in the MRT contrast agent called Resovist^{®}. From the examples of EP 1 738 774 A1 it is evident that Resovist^{®} is also suitable for MPI.

The synthesis of Multicore nanoparticles is described in "Dutz, S, J H Clement, D Eberbeck, T Gelbrich, R Hergt, R MOller, J Wotschadlo, and Zeisberger. "Ferrofluids of Magnetic Multicore Nanoparticles for Biomedical Applications." Journal of magnetism and magnetic materials 321, no. 10 (2009): doi:10.1016/j.jmmm.2009.02.073. It revealed that the dispersions which have been prepared according to the recipe given in this publication do not show a good stability.

Iron oxid nanoparticle preparations are also described for therapeutic hyperthermia. Therapeutic passive partial-body hyperthermia involves targeted incorporation of iron oxide-containing particle dispersions in tumors or tumor cells and heating by strong magnetic fields, thereby either directly damaging the tumor cells and/or increasing the effectiveness of administered chemotherapeutic agents. The use of particle dispersions containing iron oxides for therapeutic passive partial-body hyperthermia has been described for instance in WO 2006/125452. The strong magnetic fields being used not only cause heating of the particles, but also give rise to strong heating of metal-containing implants. Metallic dentures must therefore be removed from the patients prior to treatment.

It was the object of the present invention to provide a stable magnetic particle dispersion with improved nonlinear magnetization behavior and improved heating properties in alternating magnetic fields in comparison to dispersions or suspensions described in the prior art. The dispersion of the present invention should be especially useful for both magnetic particle imaging (NIPI) and therapeutic hyperthermia. In addition the dispersion of the present invention should also be useful for magnetic resonance imaging (IVIRI)

The magnetic particle dispersion provided by the present invention comprises individual monocrystalline and/or polycrystalline nanoparticles of iron oxides and nanoparticulate aggregates (multi-core particles) thereof, wherein nanoparticles and nanoparticulate aggregates are coated with a pharmaceutically acceptable coating material selected from the group comprising a synthetic polymer, a carboxylic acid or hydroxycarboxylic acid, a monosaccharid, a disaccharid, a polysaccharid, or mixtures thereof. The dispersion shows nonlinear magnetization behaviour when subjected to an alternating magnetic field and at an incident fundamental frequency of 25 kHz and 10 mT flux density and 36,6°C the value of the amplitude of the magnetic moment Aₖ generated by the dispersion and measured with the magnetic particle spectrometer ranges at 3rd harmonic from 0,3104500 to 0,3631403 Am²/mol Fe, at the 21th harmonic from 3,78193·10⁻⁴ to 4,035128·10⁻⁴ Am²/mol Fe and at the 51th harmonic from 3,933704·10⁻⁶ to 7,339437·10⁻⁶ Am²/mol Fe.

For detection of the magnetic particle dispersion of the present invention by MPI, fields from 0.1mT to 1T and frequencies from 1mHz to 1MHz can be used.

According to one embodiment of the present invention the pharmaceutically acceptable coating material can be a synthetic polymer or copolymer selected from the group consisting of polyethylenglycoles, polypropylenglycoles, polyoxyethylen and derivatives therefrom, polyoxypropylen and derivatives therefrom, polyamino acids, lactic and glycolic acid copolymers, or their mixtures.

According to another preferred embodiment of the present invention the pharmaceutically acceptable coating material is a polysaccharide selected from the group consisting of dextran, starch, chitosan, glucosaminoglycans (GAGs), starch phosphate, their derivatives, or mixtures thereof. Dextran and dextran derivatives are especially preferred. The dextran derivates are selected from the group consisting of dextran with carboxy groups, dextran with aldehyde groups, biotinylated dextran, dextran with SH-groups, reduced dextran, reduced carboxymethyldextran or mixtures thereof. In an especially preferred embodiment the coating material is carboxymethyldextran (CMD). Examples of GAGs which can be used as coating material according to the present invention include e.g. chondroitinsulfate, heparin, hyaluronan.

In another preferred embodiment of the invention as pharmaceutically acceptable coating material a monosaccharide selected from the group consisting of D-mannitol, glucose, D-mannose, Fructose, Sorbitol, Inositol, their derivatives, or mixtures thereof is used, preferably D-mannitol.

According to the invention a combination of a polysaccharide as described above and a monosaccharid as described above may also be used as coating material for the nanoparticles, e.g. a combination of D-mannitol and carboxymethyldextran.

Carboxylic acids and hydroxycarboxylic acids selected from the group consisting of citric acid, malic acid, tartaric acid, gluconic acid, a fatty acid or mixtures thereof are also useful as coating materials according to the present invention. Preferably citric acid or D-gluconic acid can be used.

The nanoparticulate iron oxide crystals of the invention comprise magnetite (Fe₃O₄) and/or maghemite (γ-Fe₂O₃) and may additionally contain other iron oxides and iron mixed oxids with Mo, Cr, Mn, Co, Cu, Ni, Zn, or mixtures thereof. Preferably the iron oxide crystals of the dispersion of the present invention comprise magnetite and/or maghemite.

Stabilization of the nanoparticulate iron oxid crystals in water or organic solvents proceeds sterically and/or electrostatically as a result of the coating material surrounding the iron oxide crystals and the dispersion or suspension shows superparamagnetic to slightly ferrimagnetic behavior in magnetic fields. In a preferred embodiment of the present invention the coated iron oxid crystals are dispersed or suspended in water, preferably they are dispersed in water.

The resulting particle dispersions of the invention comprise particles of polycrystalline and/or monocrystalline nanoparticles each having a size of from 2 to 100 nm as well as aggregates thereof embedded in a matrix of the coated material. The overall size of these single and multicore particles (hydrodynamic diameter) is between 10 and 200 nm. The individual polycrystalline and/or monocrystalline cores have sizes ranging up to the monodomain-multidomain limit, that means between 10 and 50 nm. The polycrystallites and multicore particles show the property of developing reduced anisotropy compared to monocrystalline nanoparticles of same size, resulting in an improvement of the energy transfer and/or MPS signal and in improved stability of the dispersions.

A further object of the present invention is the preparation method of the magnetic particle dispersions of the present invention and the magnetic particle dispersions obtainable by this method.

The preparation of the new particle dispersions comprises five steps a) to e) consisting of
a) alkaline precipitation of green rust (ferrous/ferric hydroxids) from iron(II) salt solution with an alkaline solution
b) oxidation with oxidants to form nanoparticulate iron oxid crystals
c) optionally, purification of the particles by magnetic separation
d) coating the particles with a pharmaceutically acceptable coating material and subsequent heating to effect growth, aggregation and the size of the particles
   or
d') heating the uncoated particles to effect growth, aggregation and the size of the particles and thereafter coating the particles with a pharmaceutically acceptable coating material and subsequent heating to effect growth, aggregation and size of the particles
   and
e) fractionating the obtained particles by magnetic separation, washing them using ultrafiltration, dialysis, centrifugation and/or diafiltration until the filtrate or the supernatant has a conductivity value of less than 10 µS and re-fractionating them by magnetic separation without or after addition of alkali.

In a preferred embodiment of the invention step d) is performed, that means the particles are first coated and then heated. Here, besides the coated multi-core particles also coated single-core particles are present. It is preferred that the heating in step d) is performed for 0.5 to 36 hours, particularly for 4 to 20 hours, especially preferred for 7,5 to 15 hours, to ensure a good growth of the single-cores and aggregates. In case of step d') it is sufficient to heat the uncoated particles for 30 minutes to 60 minutes.

In a preferred embodiment of the invention the heating to effect aggregation and growth of the coated or uncoated particles according to step d) or d') is carried out at 40 to 100°C, preferably at 85 to 95 °C, most preferred at about 90 °C.

In another preferred embodiment of the invention an aqueous solution of Fe(II) chloride tetrahydrate is used as iron(II) salt solution. Another Fe(II) salt which may be preferably used is FeSO₄ or Fe(II) sulphate heptahydrate. The alkaline solution for the precipitation in step a) is preferably a NH₄OH or KOH solution. Other bases which may be used are NaOH Na₂CO₃, NaHCO₃, K₂CO₃, KHCO₃. The oxidation step b) is preferably carried out with a H₂O₂ solution, most preferred with a 5% solution. Other oxidants which may be used are pure oxygen, atmospheric oxygen, NaNO₃, NaClO₄ and NaOCl.

In a preferred embodiment of the invention the coating in step d) or d') is performed by adding the coating material at ambient temperature and stirring.

The described magnetic particle dispersions which are prepared according to the preparation method of the invention show a pronounced overtone structure surpassing known formulations in the higher harmonics when measured in a magnetic particle spectrometer. Therefore, the dispersions of the invention are potentially suitable for MPS (Magnetic Particle Imaging). As a result of the improved energy transfer, the new particle dispersions of the invention can also be used for applications in hyperthermic therapy of tumors. The new particle dispersions are easier to magnetize (more soft-magnetic) than those previously used. As a result, treatment can be performed using lower field strengths, thereby significantly reducing the side effects of the method. Improved transfer of energy from the external alternating magnetic fields to the iron oxide systems results in improved heating. Because of their good magnetic properties the described particle dispersions are also suitable for MRI applications.

Therefore, the present invention also relates to a pharmaceutical composition comprising the magnetic particle dispersion of the invention and, optionally, pharmaceutically acceptable auxiliary substances. These auxiliary substances which can be added to diagnostic or therapeutic solutions are well known to the skilled expert. Such auxiliary substances are for instance preservatives, stabilizers, detergents, carriers, flavouring agents or phospholipides to encapsulate the magnetic particles in liposomes or micelles. They can be added to the dispersions of the invention without exception, if they are compatible with the dispersions. It is preferred that the pharmaceutical composition of the invention is a stabilized colloidal solution. In a special embodiment of the invention the pharmaceutical composition can contain surfactants like phospholipides or Pluronic® to incorporate the particles of the dispersion in micelles and liposomes. These magnetoliposomes and magnetomicelles have special characteristics and can be very useful for diagnosis and therapy. Therefore, a pharmaceutical composition which comprises magnetic particles encapsulated in liposomes or micelles is also an object of the present invention.

The pharmaceutical compositions of the present invention are especially useful in tumor staging and diagnosis of diseases e.g. of liver, spleen, bone marrow, lymph nodes, cardiovascular diseases, tumors and stroke by magnetic particle imaging (MPI) or magnetic resonance imaging (MRI). They are also useful for cell tracking or in hyperthermia, especially in passive partial-body hyperthermia and in tumor therapy by hyperthermia.

Hence, the invention also concerns a method for treating a patient in need of a tumor therapy comprising administering the magnetic particle dispersion or the pharmaceutical composition of the present invention directly to the diseased tissue of the patient and applying an alternating magnetic field (AMF) to the magnetic particle dispersion to inductively heat the magnetic particles. The magnetic particle dispersion can also be a component of an embolic agent or a mixture of embolic- and chemotherapeutic agent and administered by blood supply via a catheter.

The pharmaceutical composition of the present invention may be formulated for oral, parenteral, intratumoral, peritumoral, intralymphatic, in tissues, intravenous (IV), intra-arterial and intracerebral administration.

In the technical area the new particle dispersions of the invention can also be used for the manufacturing of electrets, pigments, functional coatings and for instance for final inspection in industrial production of non metal containing parts.

The following examples are offered to illustrate the preparation of the magnetic particle dispersions of the invention and their physical behaviour in an alternating magnetic field. The examples are not intended to be limiting in any respect.
**Figure 1** shows MPS measurements (odd harmonics) of some of the dispersions according to the invention in comparison to Resovist^{®}. Fig. 1 a exhibits an enlarged **Fig. 1** with Resovist^{®} and Example 1, solution 2.
**Figure 2** shows the TEM image of solution 2 of Example 4.
**Figure 3** shows the TEM image of solution 2 of Example 1.
**Figure 4** shows the TEM image of solution 2 of Example 2.

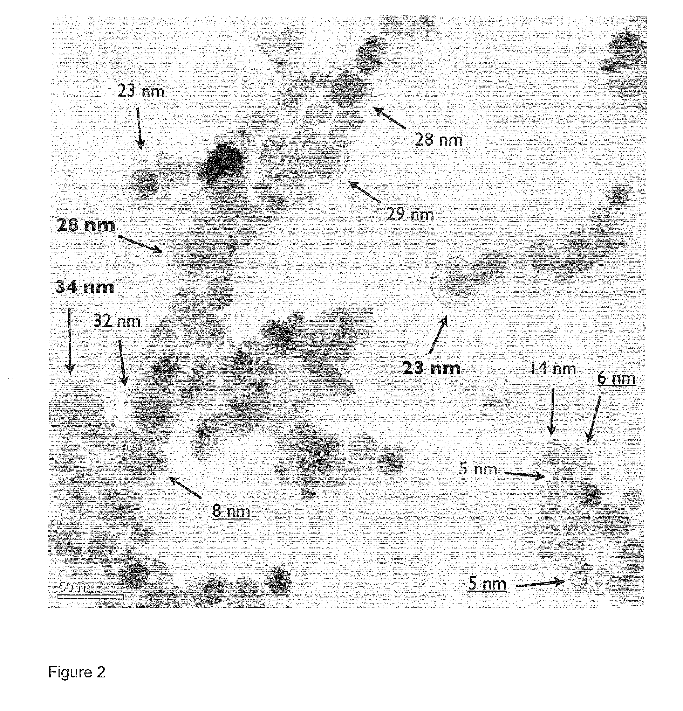

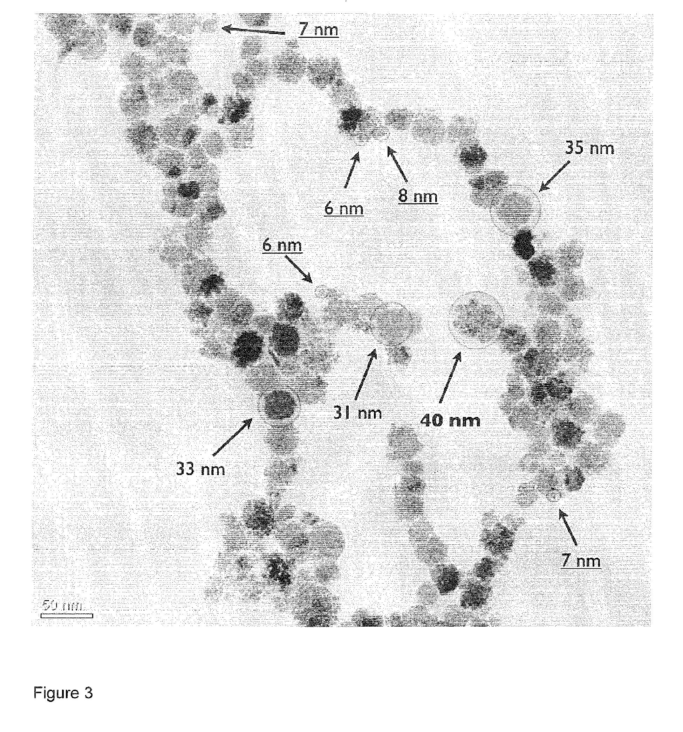

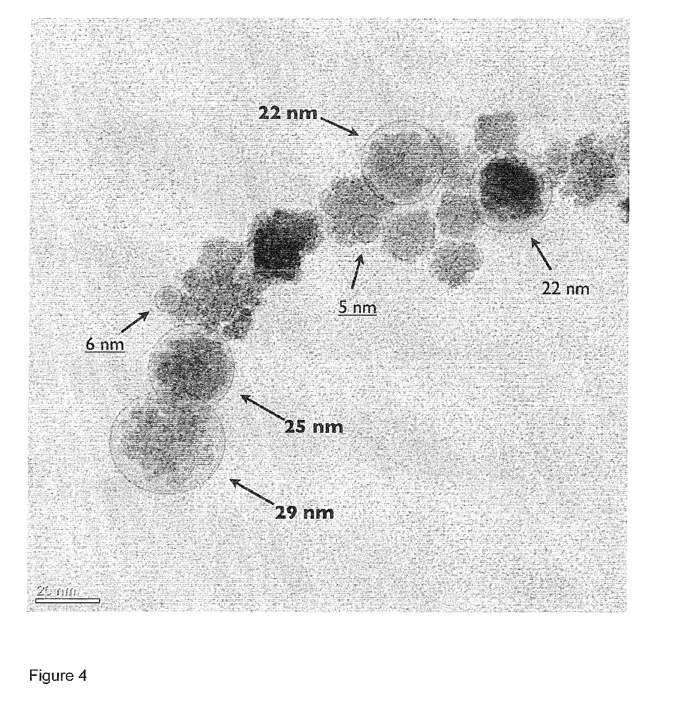

In Figures 2 to 4 the sizes of the single core particles are given in normal writing, the multicore particles are marked in fat writing and the single cores of the multicore particles are depicted underlined.

### Preparation of the magnetic particles dispersions.

### Examples 1 (NH₄OH as base, polysaccharide added)

1.90 g of Fe(II) chloride tetrahydrate is dissolved in 200 ml of water at room temperature and under an air atmosphere (20% oxygen) over a period of 5 min with stirring. Thereafter, 2 ml of ammonium hydroxide (25%) is added in one portion and stirred for about 5 min. 1 ml of hydrogen peroxide (5%) is subsequently added in one portion and the solution is stirred for 10 min. Thereafter, 4 g of carboxymethyldextran sodium salt (CMD-Na) is added and stirred for 10 min. The mixture is heated at 90°C for 600 min. Subsequently, magnetic separation is performed for 20 min, the supernatant is decanted and the sediment suspended in 200 ml of water and subjected to another magnetic separation for 20 min, the supernatant is decanted, the sediment suspended in 200 ml of water, subjected to ultrasonic treatment for 5 min and magnetic separation for 20 min, and the supenatant is decanted. The sediment can be used for further workup. The supernatants are combined and washed with water via ultrafiltration using a Vivaflow 200 filter (100 kDa RC) until the filtrate has a conductivity value of less than 10 µS and subsequently concentrated to about 25 ml. The dispersion is placed on a magnet overnight and about 25 ml (solution 1) is pipetted off to obtain solution 1. The sediment is taken up with 25 ml of water and added dropwise with 1N KOH solution until the pH of the solution is about 10. Following magnetic separation overnight, about 25 ml of solution (solution 2) is pipetted off to obtain solution 2. The sediment can be used for further workup.
**analytical data of solution 1:** iron content: 3.74 g Fe/I;
   hydrodynamic size: 21,0-37,84 nm
**analytical a of solution 2:** iron content: 0.71 g Fe/I;
   hydrodynamic size: 24,4-43,8 nm

**Example 2** (KOH as base, aggregation prior to coating and polysaccharide addition) 1.98 g of Fe(II) chloride tetrahydrate is dissolved in 200 ml of water at room temperature and under an air atmosphere (20% oxygen) over a period of 5 min with stirring. Thereafter, 22 ml of 1 N potassium hydroxide solution is added in one portion and stirred for about 5 min. 1 ml of hydrogen peroxide (5%) is subsequently added in one portion and the solution is stirred for 10 min. Subsequently, magnetic separation is performed for 5 min, the supernatant is decanted and discarded. The sediment is taken up in 100 ml of water and placed on a magnet for another 10 min. After stirring for 10 min, the suspension is heated at 90°C for 30 min and subsequently added with 4.2 g of carboxymethyldextran sodium salt (CMD-Na) and stirred for 5 min. The mixture is heated at 90°C for 420 min. Subsequently, magnetic separation is performed for 20 min, the supernatant is decanted and the sediment suspended in 200 ml of water and subjected to another magnetic separation for 20 min, the supernatant is decanted, the sediment suspended in 200 ml of water, subjected to magnetic separation for 20 min, and the supernatant is decanted, the sediment is suspended in 200 ml of water and subjected to another magnetic separation for 20 min, the supernatant is decanted and the sediment can be used for further workup. The supernatants are combined and washed with water via ultrafiltration using a Vivaflow 200 filter (100 kDa RC) until the filtrate has a conductivity value of less than 10 µS and subsequently concentrated to about 30 ml. The dispersion is placed on a magnet overnight and about 20 ml (solution 1) is pipetted off to obtain solution 1. The sediment is taken up in 25 ml of water and added dropwise with 1N KOH solution until the pH of the solution is about 10. Following magnetic separation overnight, about 23 ml of solution (solution 2) is pipetted off to obtain solution 2. The sediment is mixed with 25 ml of water and 280 mg of glycerophosphate and stirred for 5 min. Following magnetic separation overnight, about 30 ml of solution (solution 3) is pipetted off to obtain solution 3. The sediment can be used for further workup.
**analytical data of solution 1**: iron content: 2.20 g Fe/I;
   hydrodynamic size: 18,2-32,7 nm
**analytical data of solution 2**: iron content: 1.12 g Fe/I;
   hydrodynamic size: 18,2-32,7 nm
**analytical data of solution 3**: iron content: 0.48 g Fe/I;
   hydrodynamic size: 24,0-37,8 nm

### Example 3 (KOH as base, citric acid added)

1.98 g of Fe(II) chloride tetrahydrate is dissolved in 200 ml of water at room temperature and under an air atmosphere (20% oxygen) over a period of 5 min with stirring. Thereafter, 22 ml of 1 N potassium hydroxide solution is added in one portion and stirred for about 5 min. 1 ml of hydrogen peroxide (5%) is subsequently added in one portion and the solution is stirred for 10 min. Subsequently, magnetic separation is performed for 5 min, and the clear supernatant is decanted and discarded. The sediment is taken up in 50 ml of water and added with 1.1 g of citric acid monohydrate and stirred for 10 min at room temperature. The mixture is diluted to 90 ml with water and heated at 90°C for 90 min. Subsequently, magnetic separation is performed for 10 min, the supernatant is decanted and the sediment suspended in 100 ml of water and subjected to another magnetic separation for 10 min, the supernatant is decanted, the sediment suspended in 100 ml of water, subjected to magnetic separation for 10 min, and the supernatant is decanted. The sediment can be used for further workup. The supernatants are combined and washed with water via ultrafiltration using a Vivaflow 200 filter (30 kDa PES) until the filtrate has a conductivity value of less than 10 µS and subsequently concentrated to about 30 ml. The dispersion is placed on a magnet overnight and about 20 ml (solution 1) is pipetted off to obtain solution 1. The sediment is taken up with 25 ml of water and added dropwise with 1N KOH solution until the pH of the solution is about 11. Following magnetic separation overnight, about 20 ml of solution (solution 2) is pipetted off to obtain solution 2. The sediment can be used for further workup.
**analytical data of solution 1**: iron content: 0.78 g Fe/I;
   hydrodynamic size: 7,5-15,7 nm
**analytical data of solution 2**: iron content: 0.56 g Fe/I;
   hydrodynamic size: 11,7-21,0 nm

### Example 4 (KOH as base, polysaccharide added)

3.96 g of Fe(II) chloride tetrahydrate is dissolved in 200 ml of water at room temperature and under an air atmosphere (20% oxygen) over a period of 5 min with stirring. Thereafter, 44 ml of 1N potassium hydroxide solution is added in one portion and stirred for about 10 min. 2 ml of hydrogen peroxide (5%) is subsequently added in one portion and the solution is stirred for 10 min. Subsequently, magnetic separation is performed for 5 min, and the supernatant is decanted and discarded. The sediment is taken up in 200 ml of water and placed on a magnet for another 15 min. Thereafter, 8 g of carboxymethyldextran sodium salt (CMD-Na) is added and stirred for 10 min at room temperature. The mixture is diluted with water to make a total volume of 250 ml and heated at 90°C for 900 min. Subsequently magnetic separation with 100 ml of the resulting mixture is performed for 20 min, the supernatant is decanted and the sediment suspended in 200 ml of water and subjected to another magnetic separation for 20 min, the supernatant is decanted, the sediment suspended in 200 ml of water and subjected to magnetic separation for 20 min, the supernatant is decanted. The sediment is suspended in 200 ml of water and subjected to another magnetic separation for 20 min, the supernatant is decanted, the sediment is suspended in 200 ml of water and subjected to another magnetic separation for 20 min, the supernatant is decanted and the sediment is discarded or can be used for further workup. The supernatants are combined and washed with water via ultrafiltration using a Vivaflow 200 filter (100 kDa RC) until the filtrate has a conductivity value of less than 10 µS and subsequently concentrated to about 40 ml. The dispersion is placed on a magnet for 15 min, and about 35 ml is pipetted off (supernatant 1), the sediment (sediment 1) is preserved and supernatant 1 placed on a magnet overnight, and about 25 ml (solution 1) is pipetted off to obtain solution 1. The sediment 1 is taken up with 40 ml of water and added dropwise with 1N KOH solution until the pH of the solution is about 10. Following magnetic separation for 15 min, about 42 ml of solution is pipetted off (supernatant 2), supernatant 2 is placed on a magnet overnight, and about 40 ml (solution 2) is pipetted off to obtain solution 2. The sediment can be used for further workup.
**analytical data of solution 1**: iron content: 2.03 g Fe/I;
   hydrodynamic size: 18,2-28,2 nm
**analytical data of solution 2**: iron content: 1.05 g Fe/I;
   hydrodynamic size: 18,2-32,7 nm

### Example 5 (KOH as base, mono- and polysaccharide added)

3.96 g of Fe(II) chloride tetrahydrate is dissolved in 200 ml of water at room temperature and under an air atmosphere (20% oxygen) over a period of 5 min with stirring. Thereafter, 44 ml of 1N potassium hydroxide solution is added in one portion and stirred for about 5 min. 2 ml of hydrogen peroxide (5%) is subsequently added in one portion and the solution is stirred for 10 min. Subsequently, magnetic separation is performed for 12 min, and the clear supernatant is decanted and discarded. The sediment is taken up in 200 ml of water and placed on a magnet for another 15 min. Thereafter, 8 g of D-mannitol is added and stirred for 10 min at room temperature. The mixture is diluted with water to make a total volume of 250 ml and heated at 90°C for 240 min. 150 ml of this mixture, while still hot, is placed on a magnet for 15 min and subsequently decanted. The sediment is taken up in 100 ml of water and added with 4.8 g of carboxymethyldextran sodium salt (CMD-Na) and stirred for 10 min. The mixture is heated at 90°C for 510 min. Subsequently, magnetic separation is performed for 20 min, the supernatant is decanted and the sediment suspended in 200 ml of water and subjected to another magnetic separation for 20 min, the supernatant is decanted, the sediment suspended in 200 ml of water and subjected to magnetic separation for 20 min, and the supernatant is decanted. The sediment can be used for further workup. The supernatants are combined and washed with water via ultrafiltration using a Vivaflow 200 filter (100 kDa RC) until the filtrate has a conductivity value of less than 10 µS and subsequently concentrated to about 40 ml. The sediment can be used for further workup.
**analytical data**:
   iron content: 6.25 g Fe/I

### Example 6 (KOH as base, monosaccharide added)

1.98 g of Fe(II) chloride tetrahydrate is dissolved in 50 ml of water at room temperature and under an air atmosphere (20% oxygen) over a period of 5 min with stirring. Thereafter, 22 ml of 1N potassium hydroxide solution is added in one portion and stirred for about 5 min. 1 ml of hydrogen peroxide (5%) is subsequently added in one portion and the solution is stirred for 10 min. Subsequently, 4 g of D-gluconic acid sodium salt is added and stirred for 10 min at room temperature. The mixture is heated at 90°C for 240 min. The mixture is added with 1 N KOH solution until the pH of the solution is about 10. Subsequently, magnetic separation is performed for 20 min, the supernatant is decanted and the sediment suspended in 100 ml of water and subjected to another magnetic separation for 20 min, the supernatant is decanted, the sediment suspended in 100 ml of water and subjected to magnetic separation for 20 min, and the supernatant is decanted. The sediment is suspended in 100 ml of water and subjected to another magnetic separation for 20 min, and the supernatant is decanted. The sediment can be used for further workup. The supernatants are combined and washed with water via ultrafiltration using a Vivaflow 200 filter (100 kDa RC) until the filtrate has a conductivity value of less than 10 µS and subsequently concentrated to about 40 ml. The sediment can be used for further workup.
**analytical data:**
   iron content: 4.58 g Fe/I

### Example 7 (KOH as base, polysaccharide added)

3.96 g of Fe(II) chloride tetrahydrate is dissolved in 200 ml of water at room temperature and under an air atmosphere (20% oxygen) over a period of 5 min with stirring. Thereafter, 44 ml of 1N potassium hydroxide solution is added in one portion and stirred for about 10 min. 2 ml of hydrogen peroxide (5%) is subsequently added in one portion and the solution is stirred for 10 min. Subsequently, magnetic separation is performed for 12 min, and the supernatant is decanted and discarded. The sediment is taken up in 200 ml of water and placed on a magnet for another 15 min. Thereafter, 8 g of carboxymethyldextran sodium salt (CMD-Na) is added and stirred for 10 min at room temperature. The mixture is diluted with water to make a total volume of 250 ml and heated at 90°C for 450 min. Thereafter subsequently, magnetic separation is performed for 23 min, the supernatant is decanted and the sediment suspended in 165 ml of water and subjected to another magnetic separation for 23 min, the supernatant is decanted, the sediment suspended in 165 ml of water and subjected to magnetic separation for 23 min, and the supernatant is decanted. The sediment is suspended in 165 ml of water and subjected to another magnetic separation for 23 min, the supernatant is decanted, and the sediment is discarded or can be used for further workup. The supernatants are combined and washed with water via ultrafiltration using a Vivaflow 200 filter (100 kDa RC) until the filtrate has a conductivity value of less than 10 µS and subsequently concentrated to about 67 ml. The dispersion is placed on a magnet for 15 min, and about 60 ml is pipetted off (supernatant 1), the sediment (sediment 1) is preserved and supernatant 1 placed on a magnet overnight, and about 45 ml (solution 1) is pipetted off to obtain solution 1. The sediment 1 is taken up with 67 ml of water and added dropwise with 1 N KOH solution until the pH of the solution is about 10. Following magnetic separation for 15 min, about 80 ml of solution is pipetted off (supernatant 2), supernatant 2 is placed on a magnet overnight, and about 70 ml (solution 2) is pipetted off to obtain solution 2. The sediment can be used for further workup.
**analytical data of solution 1:** iron content: 5.86 g Fe/I
**analytical data of solution 2:** iron content: 1.12 g Fe/I;
   hydrodynamic size: 21,04-43,82 nm

### Comparative example 1

Multicore nanoparticles were prepared according to "Dutz, S, J H Clement, D Eberbeck, T Gelbrich, R Hergt, R Müller, J Wotschadlo, and Zeisberger. "Ferrofluids of Magnetic Multicore Nanoparticles for Biomedical Applications." Journal of magnetism and magnetic materials 321, no. 10 (2009): doi:10.1016/j.jmmm.2009.02.073.

A solution of 1M NaHCO₃ was slowly added to a FeCl₂/FoCl₃ solution (total Fe-concentration: 0.625 M; Fe²⁺/Fe³⁺ ratio = 1/1.3) with a rate of 0.75 ml/min. When the pH value reached 8 addition of the bicarbonate solution was stopped. The resulting brownish precipitate was heated to 100°C for 5min under the release of CO₂. The prepared particles were washed with deionized water three times and the pH of the resulting suspension was adjusted to pH 2-3 by the addition of diluted HCl. Then the mixture was homogenized by ultrasonic treatment for a few seconds (Sonorex Digital 10P, Bandelin electronic) and then heated to 45°C. An aqueous solution of CMD (CMD sodium salt, Fluka) with an CMD/MCNP ratio of about 1:3 was added to the suspension and stirred for 60 min at 45 °C. The coated particles were washed with de-ionized water and the resulting particle dispersion was centrifuged in a laboratory centrifuge (Labofuge 400R, Heraeus Sepatech) at 1029 x g and 20°C. The sediment was stored and the supernatant was removed. The supernatant was centrifuged again with 1525 x g. This procedure was repeated twice with 2521 x g and 2958 x g. Altogether, 8 fractions (4 sediments and 4 supernatants) were obtained.

It revealed that the magnetic properties of the obtained particle dispersions were not comparable with the one of the present invention. In addition the obtained particle dispersions showed no good stability combined with a high aggregation tendency, limiting the use as a parenteral drug.

### MPS measurements of the dispersions of the invention in comparison to Resovist^{®}

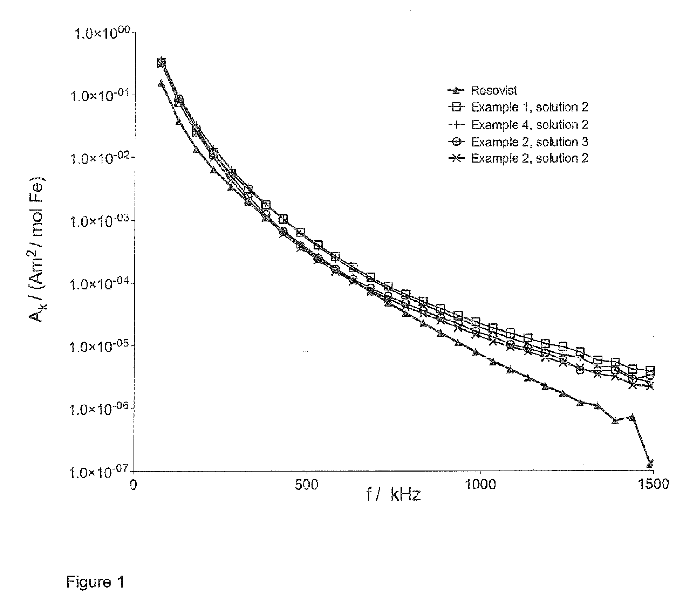

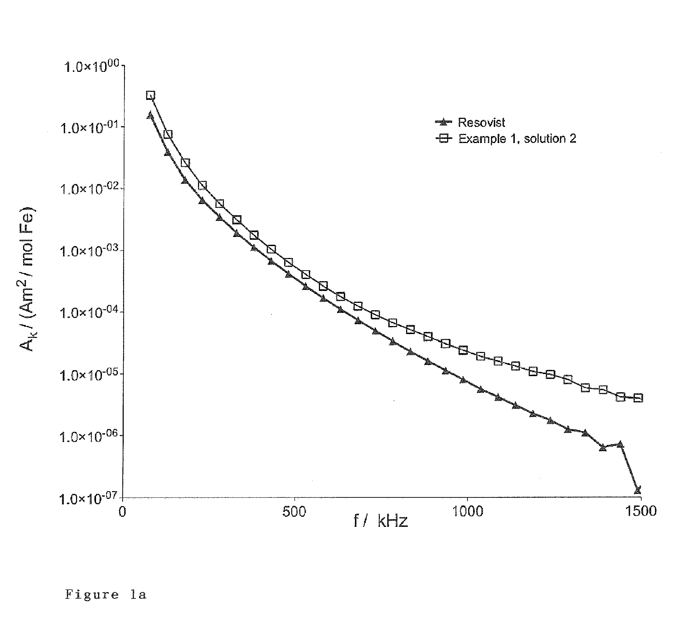

The undiluted samples of Examples 1 (solution 2), 2 (solutions 2 and 3) and 4 (solution 2) were measured in a magnetic particle spectrometer (MPS) (Bruker Biospin, Germany) at 10 mT, 25,2525 kHz, 36,6°C and for 10 s. For comparison the commercially available Resovist^{®} dispersion was diluted with water to give 100mmol Fe/L and measured under the same conditions. The measurements were carried out in PCR tubes of Life Technologies with a volume of the samples of 30µl. For evaluation the obtained measured value of each harmonic which corresponds to the amplitude of the magnetic moment was normalized to the respective iron content of each sample and given as Aₖ in Am²/mol Fe. The results are depicted in Table 1 and Figure 1. Only odd harmonics are shown.

**Table 1**

| Sample | 3rd harmonic (75,75 kHz) | 21th harmonic (530,30 kHz) | 51th harmonic (1287,88 kHz) |
|---|---|---|---|
| Example 1, solution 2 | 0,3269231 | 0,0004035128 | 7,839487*10⁻⁶ |
| Example 4, solution 2 | 0,3631403 | 0,000378193 | 6,347895*10⁻⁶ |
| Example 2, solution 3 | 0,3389185 | 0,0002457926 | 3,983704*10⁻⁶ |
| Example 2, solution 2 | 0,3104500 | 0,0002292000 | 4,353333*10⁻⁶ |
| Resovist^{®} | 0,1579576 | 0,0002615828 | 1,236485*10⁻⁶ |

As it can be taken from Table 1 the dispersions of the invention are superior to the Resovist^{®} preparation. E.g. solution 2 of Example 1 is superior to the Resovist^{®} preparation by a factor of two at the 3rd harmonic and by a factor of 6 at the 51th harmonic.

## Claims

1. A magnetic particle dispersion comprising individual monocrystalline and/or polycrystalline nanoparticles of iron oxides and nanoparticulate aggregates thereof, wherein nanoparticles and nanoparticulate aggregates are coated with a pharmaceutically acceptable coating material selected from the group comprising a synthetic polymer, a carboxylic or hydroxycarboxylic acid, a monosaccherid, a disaccharid, a polysaccharid or mixtures thereof, the dispersion showing nonlinear magnetization behaviour when subjected to an alternating magnetic field and at an incident fundamental frequency of 25,25 kHz, 10 mT flux density and 36,6°C the value of the amplitude of the magnetic moment Aₖ generated by the dispersion and measured with the magnetic particle spectrometer ranges at the third harmonic from 0,3104500 to 0,3631403 Am²/mol Fe, at the 21th harmonic from 3,78193·10⁻⁴ to 4,035128·10⁻⁴ Am²/mol Fe and at the 51th harmonic from 3,983704·10⁻⁶ to 7,839487·10⁻⁶ Am²/mol Fe.

2. The magnetic particle dispersion according to claim 1, wherein the polycrystalline and/or monocrystalline nanoparticles each have a size of from 2 to 100 nm and the overall size (hydrodynamic diameter) of these single and multicore particles is between 10 and 200 nm.

3. The magnetic particle dispersion according to claim 1 or 2, wherein the pharmaceutically acceptable coating material is a polysaccharide selected from the group consisting of dextran, starch, starch phosphate, chitosan, glucosaminoglycans and its derivatives, or mixtures thereof, preferably carboxymethyldextran.

4. The magnetic particle dispersion according to any one of claims 1 to 3, wherein the pharmaceutically acceptable coating material is a monosaccharid selected from the group consisting of D-mannitol, glucose, D-mannose, Fructose, Sorbitol, Inositol, preferably D-mannitol.

5. The magnetic particle dispersion according to any one of claims 1 to 4, wherein the pharmaceutically acceptable coating material is a carboxylic acid or hydroxycarboxylic acid selected from the group consisting of citric acid, malic acid, tartaric acid, gluconic acid, a fatty acid or mixtures thereof, preferably citric acid or D-gluconic acid.

6. The magnetic particle dispersion according to claim 1, wherein the nanoparticulate iron oxides comprise magnetite (Fe₃O₄), maghemite (γ-Fe₂O₃), iron mixed oxids with Mo, Cr, Mn, Co, Cu, Ni, Zn, or mixtures thereof, preferably magnetite and/or maghemite.

7. A method for preparing a magnetic particle dispersion according to any one of claims 1 to 6, wherein the method comprises five steps a) to e) consisting of
a) alkaline precipitation of green rust (ferrous/ferric hydroxids) from iron(II) salt solution with an alkaline solution
b) oxidation with oxidants to form nanoparticulate iron oxid crystals comprising magnetite and maghemite
c) optionally, purification of the particles by magnetic separation
d) coating the particles with a pharmaceutically acceptable coating material and subsequent heating to effect growth, aggregation and the size of the particles
or
d') heating the uncoated particles to effect growth, aggregation and the size of the particles and thereafter coating the particles with a pharmaceutically acceptable coating material and subsequent heating to effect growth, aggregation and the size of the particles
and
e) fractionating the obtained particles by magnetic separation, washing them using ultrafiltration, dialysis, centrifugation and/or diafiltration until the filtrate or the supernatant has a conductivity value of less than 10 µS and re-fractionating them by magnetic separation without or after addition of alkali.

8. The method according to claim 7, wherein an aqueous solution of Fe(II) chloride tetrahydrate or Fe(II) sulfate hepathydrate is used as iron(II) salt solution.

9. The method according to claims 7 or 8, wherein the oxidation step b) is performed with H₂O₂, pure oxygen, atmospheric oxygen, NaNO₃, NaClO₄ or NaOCl as oxidant, preferably H₂O₂.

10. The method according to any one of claims 7 to 9 , wherein the heating to effect size, aggregation and growth of the coated and uncoated particles in step d) or d') is carried out at 40 to 100 °C, preferably at 85 to 95 °C, most preferred at about 90 °C.

11. The method according to any one of claims 7 to 10, wherein the heating in step d) is performed for 0.5 to 36 hours, preferably for 4 to 20 hours, especially preferred for 7,5 to 15 hours.

12. A pharmaceutical composition comprising a magnetic particle dispersion of any one of claims 1 to 6 and pharmaceutically acceptable auxiliary substances.

13. The magnetic particle dispersion of any one of claims 1 to 6 or the pharmaceutical composition of claims 12 for use in diagnosis of diseases by magnetic particle imaging (MPI) or magnetic resonance imaging (MRI), preferably of spleen diseases, bone marrow diseases, lymph node diseases, cardiovascular diseases, tumors and stroke and for use in tumor staging.

14. The magnetic particle dispersion of any one of claims 1 to 6 or the pharmaceutical composition of claim 12 for use in cell tracking.

15. The magnetic particle dispersion of any one of claims 1 to 6 or the pharmaceutical composition of claim 12 for use in hyperthermia and tumor therapy.
